# EUROPÄISCHE PATENTSCHRIFT

(11) **EP 0 429 093 B1**
(45) Veröffentlichungstag und Bekanntmachung des Hinweises auf die Patenterteilung: **08.08.2001**
(21) Anmeldenummer: 90122957.5
(22) Anmeldetag: 08.05.1985
(51) Int. Cl.: C12N 5/10, C12N 15/82, A01H 5/00

(54) **Transformation von Pflanzenerbgut**
Transformation of plant genotype
Transformation du génotype de plantes

(30) Priorität: 11.05.1984 CH 233684; 11.02.1985 CH 60685; 01.04.1985 CH 139885
(43) Veröffentlichungstag der Anmeldung: 29.05.1991
(62) Teilanmeldung aus: 85105630.9
(73) Patentinhaber: Syngenta Participations AG, 4058 Basel (CH)
(72) Erfinder: Paszkowski, Jerzy, Dr., CH-8704 Herrliberg (CH); Potrykus, Ingo, Prof.Dr., CH-4312 Magden (CH); Hohn, Barbara, Dr., CH-4144 Arlesheim (CH); Shillito, Raymond Douglas, Dr., Chapel Hill, North Carolina 27514 (US); Hohn, Thomas, Dr., CH-4144 Arlesheim (CH); Saul, Michael William, Dr., CH-8003 Zürich (CH); Mandak, Vaclav, CH-4455 Zunzgen (CH)
(74) Vertreter: Zumstein, Fritz, Dr.

(56) Entgegenhaltungen:
- EP-A- 0 159 418
- WO-A-83/01176
- WO-A-85/01856
- Z. PFLANZENPHYSIOL., Band 94, 1979, Seiten 95-99; J. KOROHODA et al.: "High efficiency genetic transformation in maize induced by exogenous DNA"
- SCIENCE, Band 223, Februar 1984, Seiten 496-498; "Inheritance of functional foreign genes in plants"
- NATO ASI SERIES A, Band 83, Mol. Form. Funct. Plant Genome. Advanced Course, 4. - 14. Juli 1984, Seiten 479-488; W.R. HIATT et al.: "Introduction and expression in plants of a glyphosate resistant aroA gene isolated from Salmonella Typhimurium"
- JOURNAL OF CELLULAR BIOCHEMISTRY, suppl. 7B, 1983, page 268, Zusammenfassung Nr. 1211; Th. HOHN et al.: "Drug resistant plant cell lines after treatment of protoplasts with hybrid DNA"
- THE EMBO JOURNAL, Band 3, Nr. 12, 1984, Seiten 2717-2722, IRL Press Ltd, Oxford, GB; J. PASZKOWSKI et al.: "Direct gene transfer to plants"
- MOL. GEN. GENET., Band 199, Nr. 2, Mai 1985, Seiten 183-188; I. POTRYKUS et al.: "Direct gene tranfer to cells of a graminaceous mononcot"
- MANIPULATION AND EXPRESSION OF GENES IN EUKARYOTES, 1983, Seiten 241-244, ed. P. Nagley et al., Academic Press Australia; M. FISCHER et al.: "Experiments on transformation of plant cells using synthetic DNA vectors"

## Beschreibung

Die vorliegende Erfindung betrifft ein neues Verfahren zur Transformation pflanzlichen Erbgutes und die nach diesem Verfahren erhältlichen pflanzlichen Produkte.

Durch die Einführung neuer genetischer Information in pflanzliches Material können Pflanzen mit neuen und/oder verbesserten Eigenschaften erzeugt werden.

Angesichts des raschen Anstiegs der Weltbevölkerung und des damit verbundenen höheren Nahrungsmittel- und Rohstoffbedarfs gehört die Steigerung des Ertrags von Nutzpflanzen sowie die vermehrte Gewinnung von pflanzlichen Inhaltsstoffen, insbesondere der Fortschritt auf dem Gebiet der Nahrungs- und Heilmittel, zu den dringlichsten Aufgaben der biologischen Forschung. In diesem Zusammenhang sind als wesentliche Aspekte beispielsweise zu nennen: eine Erhöhung der Resistenz von Nutzpflanzen gegen ungünstige Bodenverhältnisse oder Klimabedingungen, gegen Krankheiten und Schädlinge, eine gesteigerte Resistenz gegen Pflanzenschutzmittel wie Insektizide, Herbizide, Fungizide und Bakterizide, und eine günstige Veränderung des Nährstoffgehalts oder des Ernteertrags von Pflanzen. Solche wünschenswerten Effekte könnten allgemein durch Induktion oder vermehrte Bildung von Schutzstoffen, wertvollen Proteinen oder Toxinen herbeigeführt werden. Eine entsprechende Beeinflussung von pflanzlichem Erbgut kann beispielsweise dadurch erfolgen, dass man ein bestimmtes Fremdgen gezielt in Pflanzenzellen einschleust, ohne hierfür von konventionellen züchterischen Massnahmen Gebrauch zu machen.

Die Uebertragung von neuen DNS-Sequenzen in Pflanzenzellen unter Verwendung von genetisch manipulierten Pflanzenbakterien sind aus der Literatur durch einige Publikationen, wie zum Beispiel Nature, Vol. 303, 209-213 (1983); Nature, Vol. 304, 184-187 (1983); Scientific American 248(6), 50-59 (1983); EMBO-Journal 2(6), 987-995 (1983); Science 222, 476-482 (1983); Science 223, 247-248 (1984); oder Proc. Natl. Acad. Sci. USA 80, 4803-4807 (1983) bekannt geworden. Die natürlichen pflanzeninfektiösen Eigenschaften dieser Bakterien wurden dabei ausgenutzt, um neues genetisches Material in Pflanzenzellen einzuschleusen. Zu diesem Zweck wurden bisher vorzugsweise Agrobacterium tumefaciens als solches oder dessen Ti-Plasmid eingesetzt, ferner auch Cauliflower-Mosaik-Virus.

Das neue Verfahren ermöglicht dagegen die direkte Uebertragung eines Gens ohne Einsatz biologischer Vektoren. Bei den bisher bekannten Verfahren werden als Vektoren Pathogene eingesetzt. Da das erfindungsgemässe Verfahren ohne Pathogene ausgeführt wird, entfallen somit auch die Einschränkungen, welche durch die Wirtsspezifität der Pathogene gegeben sind. Die Entwicklung der Pflanzen, an denen das neuartige Verfahren der Transformation durchgeführt wird, wird durch dieses nicht beeinträchtigt.

Neben dem erfindungsgemässen Verfahren zur Transformation pflanzlichen Erbgutes betrifft die Erfindung auch die nach diesem Verfahren erhältlichen Produkte, insbesondere Protoplasten und aus diesen hervorgegangenes pflanzliches Material, wie beispielsweise Zellen und Gewebe, insbesondere vollständige Pflanzen, welche aus den Protoplasten regeneriert worden sind, und deren genetisch identische Nachkommenschaft.

Im Rahmen der vorliegenden Erfindung gelten folgende Definitionen:

| | |
|---|---|
| Gen | Strukturgen mit flankierenden Expressionssignalen |
| Strukturgen | Protein-kodierende DNS-Sequenz |
| Expressionssignale | Promotersignal und Terminationssignal |
| Pflanzenwirksames Expressionssignal | Expressionssignal, das in Pflanzen funktioniert |
| Promotersignal | die Transkription initiierendes Signal |
| Terminationssignal | die Transkription terminierendes Signal |
| Enhancersignal | die Transkription verstärkendes Signal |
| Replikationssignal | die DNS-Replikation ermöglichendes Signal |
| Integrationssignal | DNS-Sequenz, welche die Integration des Gens in die genomische DNS fördert |
| Hybridgen | aus heterologen DNS-Sequenzen, d.h., DNS-Sequenzen verschiedenen Ursprungs aufgebautes Gen, wobei es sich sowohl um natürliche als auch um synthetische DNS-Sequenzen handeln kann |
| Träger-DNS | das Gen flankierende neutrale, d.h., an der Funktion des Gens nicht beteiligte DNS-Sequenz |
| Isoliertes Gen | aus der originären DNS herausgetrennte DNS-Sequenz, welche ein einziges Protein kodiert |
| NPT-II-Gen | Neomycin-3'-Phosphotransferase-Gen, Typ II von Transposon Tn 5 (Rothstein, S.J. und W.S. Reznikoff, Cell 23, 191-199, (1981)) |
| Genomische DNS | DNS des Pflanzengenoms (total oder Teil davon) |

Gemäss vorliegender Erfindung wird ein neues Verfahren zur Transformation pflanzlichen Erbgutes vorgeschlagen, das dadurch gekennzeichnet ist, dass man ein Gen ohne Zuhilfenahme natürlicher pflanzeninfektiöser Systeme direkt in Pflanzenzellen überträgt. Es handelt sich hierbei also um eine Vektor-freie Transformation. Bei dieser Vektor-freien Transformation steht das einzuführende Fremdgen unter Kontrolle von pflanzenwirksamen Expressionssignalen. Vorzugsweise wird die Vektor-freie Transformation pflanzlicher Gene ausgeführt, indem man ein einzuführendes Fremdgen und als Rezipienten fungierende pflanzliche Protoplasten (Empfänger-Protoplasten) gemeinsam in eine geeignete Lösung einbringt und darin bis zur Aufnahme des Gens in die Protoplasten belässt.

Als pflanzliche Protoplasten werden vorzugsweise jeweils solche einer einzigen Pflanzenart oder einer der Art untergeordneten systematischen Einheit verwendet.

Das Fremdgen und die Protoplasten werden zweckmässigerweise über einen Zeitraum von einigen Sekunden bis zu einigen Stunden, vorzugsweise 10 bis 60 Minuten, insbesondere 30 Minuten, in der Lösung belassen.

Das erfindungsgemässe Verfahren ist breit anwendbar. So kann man beliebige Strukturgene pflanzlicher Herkunft, wie beispielsweise das Zein-Gen (Wienand, U., et al., Mol.Gen.Genet. 182, 440-444 (1981)), tierischer Herkunft, wie beispielsweise das TPA-Gen (tissue-type plasminogen activator-Gen; Pennica, D., et al., Nature 301, 214-221, (1983)), mikrobieller Herkunft wie beispielsweise das NPT-II-Gen, oder auch synthetischer Herkunft, wie beispielsweise das Insulin-Gen (Stepien, P., et al., Gene 24, 289-297 (1983)), in das Erbgut von Pflanzen übertragen, unter der Voraussetzung, dass die Strukturgene von in Pflanzen Expression bewirkenden Expressionssignalen flankiert sind, wobei die Expressionssignale pflanzlicher, tierischer, mikrobieller oder synthetischer Herkunft sein können.

Die übertragenen Gene, bestehend aus Strukturgen und flankierenden Expressionssignalen, können dabei sowohl natürlich vorkommende Gene als auch Hybridgene sein. Bevorzugt werden im erfindungsgemässen Verfahren diejenigen Gene eingesetzt, deren Expressionssignale tierischer oder insbesondere pflanzlicher oder synthetischer Herkunft sind. Beispiele für derartige Gene sind:
a) vollständige Gene aus Pflanzen, bestehend aus dem Strukturgen mit seinen natürlichen Expressionssignalen;
b) vollsynthetische Gene, bestehend aus einem Strukturgen synthetischer Herkunft, flankiert von Expressionssignalen synthetischer Herkunft;
c) Strukturgene pflanzlicher Herkunft, flankiert von pflanzenwirksamen Expressionssignalen, wobei Strukturen und Expressionssignale von verschiedenen Pflanzenarten stammen;
d) Strukturgene pflanzlicher Herkunft, flankiert von Expressionssignalen synthetischer Herkunft;
e) Strukturgene tierischer, mikrobieller oder synthetischer Herkunft, flankiert von Expressionssignalen pflanzlicher Herkunft; oder
f) Strukturgene tierischer oder mikrobieller Herkunft, flankiert von Expressionssignalen synthetischer Herkunft.

Ganz besonders bevorzugt sind Strukturgene bakterieller Herkunft, flankiert von Expressionssignalen pflanzlicher Herkunft, insbesonsondere solchen mit Ursprung aus Pflanzenviren. Als bei der Anwendung gemäss vorliegender Erfindung besonders geeignete Expressionssignale haben sich die Expressionssignale des Gens VI des Cauliflower-Mosaik-Virus erwiesen.

Die Hybridgene werden nach an sich bekannten mikrobiologischen Verfahren hergestellt, wobei das Leseraster der Kodierung für die von der Pflanzenzelle zu produzierenden Proteine beibehalten wird. Derartige Methoden sind bekannt und werden beispielsweise in folgenden Publikationen beschrieben: "Molecular Cloning", Maniatis, T., Fritsch, E.F. und J. Sambrook, Cold Spring Harbor Laboratory, 1982, und "Recombinant DNA Techniques", Rodriguez, R.L. und R.C. Tait, Addison-Wesley Publishing Comp., London, Amsterdam, Don Mills. Ontario, Sydney, Tokyo, 1983.

Für die Integration des Fremdgens in die genomische DNS der Pflanzenzelle ist es vorteilhaft, wenn das Gen, bestehend aus Strukturgen und pflanzenwirksamen Expressionssignalen, von neutralen DNS-Sequenzen (Träger-DNS) flankiert wird. Die Träger-DNS kann dabei aus zwei linearen DNS-Strängen bestehen, so dass die in die Pflanzenzelle einzuschleusende Konstruktion ein lineares DNS-Molekül ist. Die zur Genübertragung hergestellte DNS-Sequenz kann aber auch eine ringförmige Struktur (Plasmidstruktur) haben. Solche Plasmide bestehen aus einem DNS-Strang, in den das Fremdgen mit den Expressionssignalen integriert ist. Die Träger-DNS kann synthetischen Ursprungs sein oder durch Behandlung mit geeigneten Restriktionsenzymen aus natürlich vorkommenden DNS-Sequenzen gewonnen werden. So sind beispielsweise als Träger-DNS natürlich vorkommende Plasmide geeignet, die mit einem selektiven Restriktionsenzym geöffnet worden sind.

Ein Beispiel für ein derartiges Plasmid ist das frei erhältliche Plasmid pUC8 (beschrieben in Messing, J. und J. Vieira, Gene 19, 269-276, 1982). Weiter sind als Träger-DNS auch Bruchstücke natürlich vorkommender Plasmide verwendbar. Beispielsweise ist als Träger-DNS die Deletionsmutante für Gen VI des Cauliflower-Mosaik-Virus einsetzbar.

Die Wahrscheinlichkeit der genetischen Transformation einer Pflanzenzelle kann durch verschiedene Faktoren erhöht werden. So steigt, wie man aus Versuchen mit Hefe weiss, die Anzahl der erfolgreichen, stabilen Gentransformationen
1) mit steigender Anzahl von Kopien der neuen Gene pro Zelle,
2) bei Kombination eines Replikationssignals mit dem neuen Gen, sowie
3) bei Kombination eines Integrationssignals mit dem neuen Gen.

Somit ist das erfindungsgemässe Verfahren besonders vorteilhaft anzuwenden, wenn das übertragene Gen mit einem in Pflanzenzellen wirksamen Replikations- oder einem in Pflanzenzellen wirksamen Integrationssignal oder mit beiden Signalen kombiniert ist.

Die Expression eines Gens in einer Pflanzenzelle ist abhängig von der Transkriptionshäufigkeit des Gens in eine Messenger-RNS-Sequenz. Es ist daher ebenfalls von Vorteil, wenn das neue Gen mit einem diese Transkription verstärkenden Enhancersignal kombiniert ist. Insbesondere sind diejenigen Verfahren hervorzuheben, bei denen ein Gen übertragen wird, das mit in Pflanzenzellen wirksamen Replikations-, Integrations- und Enhancersignalen kombiniert ist.

Weiterhin ist es von grossem verfahrenstechnischem Vorteil, wenn das übertragene Gen eine selektive Markerfunktion besitzt, d.h., dass die transformierten Pflanzenzellen von den nicht-transformierten Pflanzenzellen durch eine gezielte Selektionierung getrennt werden können. Eine derartige Markerfunktion erlaubt eine rationelle Arbeitsweise dadurch, dass nur diejenigen Pflanzenzellen durch übliche mikrobiologische Massnahmen zu Kalli oder vollständigen Pflanzen regeneriert werden müssen, in deren Erbgut ein zur Expression gelangendes Gen vorhanden ist, welches Marker-spezifische Selektionierungsmassnahmen gestattet.

Während als Pflanzenzellen, die als Ausgangsmaterialien für eine Transformation in Betracht zu ziehen sind, Protoplasten, Zellkulturzellen, Zellen in Pflanzengeweben, Pollen, Pollenschläuche, Eizellen, Embryosäcke oder Zygoten und Embryonen in unterschiedlichen Entwicklungsstadien zu nennen sind, sind Protoplasten wegen der direkten Einsatzmöglichkeit ohne weitere Vorbehandlungen bevorzugt.

Die Gewinnung pflanzlicher, isolierter Protoplasten, Zellen oder Gewebe kann nach an sich bekannten Methoden oder analog dazu erfolgen.

Isolierte pflanzliche Protoplasten, welche sich auch als Ausgangsmaterial für isolierte Zellen und Gewebe eignen, können von beliebigen Teilen der Pflanze gewonnen werden, wie beispielsweise von Blättern, Keimlingen, Stengeln, Blüten, Wurzeln oder Pollen. Bevorzugt werden Blattprotoplasten verwendet. Die isolierten Protoplasten können auch aus Zellkulturen gewonnen werden. Methoden für die Isolierung von Protoplasten finden sich beispielsweise in Gamborg, O.L. und Wetter, L.R., Plant Tissue Culture Methods, 1975, 11-21.

Die Uebertragung der neuen Gene in die Pflanzenzellen erfolgt auf direktem Wege ohne Benützung eines natürlichen pflanzeninfektiösen Systems, wie eines Pflanzenbakteriums, eines Pflanzenvirus oder Uebertragung durch Insekten oder phytopathogene Pilze. Zu diesem Zweck werden die zu transformierenden Pflanzenzellen direkt mit dem zu übertragenden Gen behandelt, indem man das Fremdgen und pflanzliche Protoplasten in eine geeignete Lösung einbringt und darin über eine Zeitspanne, welche für die Aufnahme des Fremdgens in die Protoplasten ausreicht, belässt. Durch die Kombination dieser Massnahme mit Techniken, die in der mikrobiologischen Forschung zur Genübertragung angewendet werden, wie beispielsweise Poly-L-Ornithin- oder Poly-L-Lysin-Behandlung, Liposomenfusion, DNS-Proteinkomplexierung, Ladungsänderung an der Protoplastenmembran, Fusion mit mikrobiellen Protoplasten oder Calciumphosphat-Copräzipitation, und insbesondere Polyäthylenglykolbehandlung, Hitzeschock-Methode (heat shock) und Elektroporation sowie Kombination dieser drei letztgenannten Massnahmen untereinander, lässt sich die Transformationshäufigkeit steigern.

Als Lösungen, in welche das Fremdgen und die Empfängerprotoplasten eingeführt werden, kommen vorzugsweise osmotisch stabilisierte Kulturmedien, wie sie für Protoplastenkulturen verwendet werden, in Betracht.

Es stehen bereits zahlreiche Kulturmedien zur Verfügung, die sich in einzelnen Medienkomponenten oder Gruppen solcher Komponenten unterscheiden. Alle Medien sind jedoch nach dem folgenden Prinzip aufgebaut: sie enthalten eine Gruppe anorganischer Ionen im Konzentrationsbereich von ca. 10 mg/l bis zu einigen Hundert mg/l (sogenannte Makroelemente wie beispielsweise Nitrat, Phosphat, Sulfat, Kalium, Magnesium, Eisen), eine weitere Gruppe anorganischer Ionen in Mengen von maximal einigen mg/l (die sogenannten Mikroelemente wie beispielsweise Kobalt, Zink, Kupfer, Mangan), ferner eine Reihe von Vitaminen (beispielsweise Inosit, Folsäure, Thiamin), eine Energie- und Kohlenstoffquelle wie beispielsweise Saccharose oder Glukose, sowie Wachstumsregulatoren in Form natürlicher oder synthetischer Phytohormone aus den Klassen der Auxine und Cytokinine im Konzentrationsbereich von 0,01 bis 10 mg/l. Die Kulturmedien sind zudem osmotisch stabilisiert durch Zusatz von Zuckeralkoholen (beispielsweise Mannit) oder Zucker (beispielsweise Glukose) oder Salzionen (beispielsweise CaCl₂) und sind auf einen pH-Wert von 5,6 bis 6,5 eingestellt.

Eine nähere Beschreibung gängiger Kulturmedien findet sich beispielsweise in Koblitz, H., Methodische Aspekte der Zell- und Gewebezüchtung bei Gramineen unter besonderer Berücksichtigung der Getreide, Kulturpflanze XXII, 1974, 93-157.

Als Technik zur Gentransformation ist besonders die "Polyäthylenglykolbehandlung" geeignet, wobei im Rahmen der vorliegenden Erfindung der Begriff "Polyäthylenglykol" nicht nur für die Substanz Polyäthylenglykol selbst steht, sondern als Oberbegriff für alle Substanzen, die ebenfalls die Protoplastenmembran modifizieren und wie sie beispielsweise auf dem Gebiet der Zellfusion zum Einsatz gelangen, zu verstehen ist. Der Begriff umfasst somit beispielsweise auch andere längerkettige, mehrwertige Alkohole, wie Polypropylenglykol (425 bis 4000 g/Mol), Polyvinylalkohol oder mehrwertige Alkohole, deren Hydroxygruppen teilweise oder vollständig veräthert sind, sowie pflanzenverträgliche, auf dem Agrargebiet gebräuchliche Detergentien, wie sie beispielsweise in folgenden Publikationen beschrieben werden:
"Mc Cutcheon's Detergents and Emulsifiers Annual"
MC Publishing Corp., Ridgewood New Jersy, 1981;
Stache, H., "Tensid-Taschenbuch",
Carl Hanser Verlag, München/Wien, 1981

Sofern Polyäthylenglykol selbst eingesetzt wird (wie in den Beispielen 1 bis 3, 5 und 7), verwendet man vorzugsweise solches mit einem Molekulargewicht zwischen 1000 und 10'000 g/Mol, insbesondere zwischen 3000 und 8000 g/Mol.

Von den vorgenannten Mitteln ist die Substanz Polyäthylenglykol selbst bevorzugt.

Mit den vorgenannten Massnahmen lässt sich bereits eine beachtliche und reproduzierbare Transformationshäufigkeit von 10⁻⁵ erzielen, welche jedoch durch geeignete Massnahmen, wie sie nachfolgend näher erläutert werden, noch wesentlich verbessert werden kann.

Bei der Polyäthylenglykolbehandlung kann man beispielsweise so vorgehen, dass man entweder eine Suspension der Protoplasten in einem Kulturmedium vorlegt und anschliessend das Gen, welches gewöhnlich als Plasmid eingesetzt wird, in einem Gemisch aus Polyäthylenglykol und Kulturmedium zusetzt, oder vorteilhafterweise Protoplasten und Gen (Plasmid) im Kulturmedium vorlegt und dann erst Polyäthylenglykol zusetzt.

Im Rahmen des erfindungsgemässen Verfahrens haben sich ferner als besonders vorteilhafte Massnahmen die Elektroporation und die Hitzeschockbehandlung erwiesen.

Bei der Elektroporation (Neumann, E. et al., The EMBO Journal 7, 841-845 (1982)) werden Protoplasten in einem Osmoticum, beispielsweise einer Mannit/Magnesium-Lösung suspendiert und die Protoplastensuspension wird in die zwischen zwei Elektroden befindliche Elektroporatorkammer eingebracht. Die Protoplasten werden nun durch Entladen eines Kondensators über die Suspensionsflüssigkeit einem elektrischen Impuls von hoher Spannung und von kurzer Dauer ausgesetzt. Hierdurch wird eine Polarisierung der Protoplastenmembran und die Oeffnung von Poren in der Membran bewirkt.

Bei der Hitzebehandlung werden Protoplasten in einem Osmoticum, beispielsweise einer Mannit/Calciumchlorid-Lösung, suspendiert und in kleinen Behältern, wie beispielsweise Zentrifugenröhrchen, erhitzt, zweckmässigerweise im Wasserbad. Die Dauer der Erhitzung richtet sich nach der gewählten Temperatur. Im allgemeinen liegen die Werte im Bereich von 40°C während einer Stunde und 80°C während einer Sekunde. Optimale Ergebnisse liefert eine Temperatur von 45°C über eine Dauer von 5 Minuten. Anschliessend wird die Suspension auf Raumtemperatur oder tiefer abgekühlt.

Es hat sich ferner gezeigt, dass die Transformationshäufigkeit durch Inaktivierung der extrazellulären Nukleasen erhöht werden kann. Eine solche Inaktivierung kann durch den Einsatz pflanzenverträglicher zweiwertiger Kationen, wie beispielsweise Magnesium oder Calcium, sowie vorzugsweise auch durch Vornahme der Transformation bei hohem pH-Wert, wobei als optimaler Bereich ein pH von 9 bis 10,5 anzusehen ist, erfolgen.

Ueberraschenderweise resultiert aus dem gezielten Einsatz dieser verschiedenen Massnahmen eine enorme Steigerung der Transformationshäufigkeit, wie sie auf dem Gebiet der Gentechnologie seit langem angestrebt wird.

Je geringer bei einer Gentransformation die Transformationshäufigkeit ist, desto schwieriger und aufwendiger ist das Auffinden der wenigen, aus den transformierten Zellen resultierenden Zellklone unter der enormen Zahl nicht transformierter Klone, und die Anwendung üblicher Screening-Methoden ist bei geringer Transformationshäufigkeit nahezu oder gänzlich unmöglich, es sei denn, dass es sich um ein Gen mit selektiver Marker-Funktion handelt (z.B. Resistenz gegen eine bestimmte Substanz). Geringe Transformationshäufigkeit erfordert also bei Genen ohne Marker-Funktion einen enormen Aufwand.

Bei Transformationen mit Genen ohne Markerfunktion lassen sich übliche Screening-Methoden zur Selektionierung transformierter Zellklone erst dann sinnvoll und erfolgreich einsetzen, wenn die Transformationshäufigkeit in der Grössenordnung von Prozenten (ca. 10⁻²) liegt. Wie nachstehend gezeigt wird, lässt sich die angestrebte Transformationshäufigkeit mit dem erfindungsgemässen Verfahren nunmehr erreichen. Ueberraschenderweise resultiert aus dem gezielten Einsatz verschiedener Massnahmen im Rahmen des erfindungsgemässen Verfahrens eine enorme Steigerung der bisher erreichten Transformationshäufigkeit bis auf 1 bis 2 %.

Die Zusammenführung von Fremdgen und Empfänger-Protoplasten vor den übrigen Massnahmen wie beispielsweise Polyäthylenglykolbehandlung, Elektroporation und Hitzeschockbehandlung, bewirkt gegenüber einem Vorgehen in anderer Reihenfolge eine Verbesserung der Transformationshäufigkeit um etwa eine Zehnerpotenz.

Durch Elektroporation lässt sich eine Verbesserung der Transformationshäufigkeit um das 5- bis 10-fache und durch Hitzeschockbehandlung eine solche um das Zehnfache oder mehr erreichen.

Als vorteilhaft erweist sich eine Kombination von zwei oder drei der Techniken: Polyäthylenglykolbehandlung, Hitzeschockbehandlung und Elektroporation, wobei besonders gute Ergebnisse erzielt werden, wenn diese Techniken erst nach Einbringen von Fremdgen und Protoplasten in eine Lösung angewendet werden. Bevorzugt ist die Anwendung der Hitzeschockbehandlung vor der Polyäthylenglykol-behandlung und der gegebenenfalls noch nachfolgenden Elektroporation. Im allgemeinen bringt die zusätzliche Anwendung der Elektroporation noch eine weitere Steigerung der Transformationshäufigkeit, in einigen Fällen werden die durch Hitzeschock- und Polyäthylenglykolbehandlung erzielten Resultate durch zusätzliche Elektroporation jedoch nicht mehr wesentlich verbessert.

Ebenso wie die Techniken untereinander lässt sich auch der Einsatz pflanzenverträglicher zweiwertiger Kationen und/oder die Durchführung der Transformation bei einem pH von 9 bis 10,5 sowohl mit einzelnen Techniken als auch mit Kombinationen von Techniken, insbesondere mit Polyäthylenglykolbehandlung, Hitzeschockbehandlung und Elektroporation, kombinieren. Durch die zahlreichen Variationsmöglichkeiten lässt sich das erfindungsgemässe Verfahren den jeweiligen Bedingungen ausgezeichnet anpassen.

Die Kombination von Hitzeschockbehandlung, Polyäthylenglykol-behandlung und gegebenenfalls Elektroporation im Anschluss an die bereits erfolgte Zusammenführung von Fremdgen und Empfänger-Protoplasten führt nun zu einer Transformationshäufigkeit von 10⁻² bis 10⁻³.

Mit dem erfindungsgemässen Verfahren lässt sich also eine hohe Transformationshäufigkeit erreichen, und zwar ohne Einsatz biologischer Vektoren für die Transformation, wie beispielsweise Cauliflower-Mosaik-Virus oder Agrobacterium.

Eine vorteilhafte Methode besteht beispielsweise darin, dass man Protoplasten in eine Mannit-Lösung überführt und die so erhaltene Protoplastensuspension mit der wässrigen Genlösung vermischt. Anschliessend werden die Protoplasten in dieser Mischung 5 Minuten lang bei 45°C inkubiert und dann innerhalb 10 Sekunden auf 0°C abgekühlt. Anschliessend wird Polyäthylenglykol (MW 3000 bis 8000) zugegeben, bis eine Konzentration von 1 bis 25 %, bevorzugt von etwa 8 %, erreicht ist. Nach sorgfältiger Durchmischung erfolgt die Behandlung im Elektroporator. Anschliessend wird die Protoplasten-suspension mit Kulturmedium verdünnt und die Protoplasten werden in Kultur genommen.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Bromeliaceae, Rubiaceae, Theaceae, Musaceae oder Gramineae und der Ordnung Leguminosae und hier vor allem der Familie Papilionaceae. Bevorzugt sind Vertreter der Familien Solanaceae, Cruciferae und Gramineae.

Besonders erwähnenswert sind Pflanzen der Gattungen Nicotiana, Petunia, Hyoscyamus, Brassica und Lolium, wie beispielsweise Nicotiana tabacum, Nicotiana plumbagenifolia, Petunia hybrida, Hyoscyamus muticus, Brassica napus, Brassica rapa und Lolium multiflorum.

Die Kulturpflanzen mit grossen Ernteerträgen wie Mais, Reis, Weizen, Gerste, Roggen, Hafer oder Hirse sind in erster Linie Objekt der Anstrengungen auf dem Gebiet der Transformation von Pflanzenzellen.

Alle Pflanzen, die durch Regeneration aus Protoplasten erzeugt werden können, lassen sich auch unter Anwendung des erfindungsgemässen Verfahrens transformieren. Vertreter der Familie Gramineae (Gräser), welche auch Getreide umfasst, konnten bisher nicht genetisch manipuliert werden. Es wurde nunmehr nachgewiesen, dass mit der vorstehend beschriebenen Methode der direkten Gentransformation auch Gramineenzellen, also auch Getreidezellen, genetisch transformiert werden können. In gleicher Weise ist eine Transformation der Kulturpflanzen der Gattungen Solanum, Nicotiana, Brassica, Beta, Pisum, Phaseolus, Glycine, Helianthus, Allium, Weizen, Gerste, Hafer, Setaria, Raps, Reis, Cydonia, Pyrus, Malus, Rubus, Fragaria, Prunus, Arachis, Secale, Panicum, Saccharum, Coffea, Camellia, Musa, Ananas, Vitis oder Citrus möglich und erwünscht, wenn auch die Gesamterträge und -anbauflächen hierfür weltweit geringer sind.

Der Nachweis transformierter Gene kann auf an sich bekannte Weise erfolgen, beispielsweise durch Kreuzungsanalysen und molekularbiologische Untersuchungen, zu denen besonders die "Southern blot"-Analyse und Enzymaktivitätstests gehören.

Die "Southern blot"-Analyse kann beispielsweise folgendermassen durchgeführt werden: Die den transformierten Zellen oder Protoplasten entnommene DNS wird nach Behandlung mit Restriktionsenzymen einer Elektrophorese in 1 %-igem Agarose-Gel unterworfen, auf eine Nitrozellulosemembran (Southern, E.M., J.Mol.Biol. 98, 503-517 (1975)) transferiert und mit der nachzuweisenden DNS, welche einer Nick-Translation (Rigby, W.J., Dieckmann, M., Rhodes, C.und P.Berg, J.Mol. Biol. 113, 237-251, (1977)) unterworfen wurde (DNS-spezifische Aktivität von 5 x 10⁸ bis 10 x 10⁸ c.p.m./µg) hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNS wird durch Schwärzung eines Röntgenfilms während 24 bis 48 Stunden sichtbar gemacht.

Eine Untersuchung auf Enzymaktivität lässt sich - näher erläutert am Test auf Aminoglykosidphosphotransferase (Enzym für Kanamycinspezifische Phosphorylierung) - beispielsweise folgendermassen durchführen: Kallus- oder Blattstücke (100 bis 200 mg) werden in 20 µl Extraktionspuffer in einem Eppendorf-Zentrifugenröhrchen homogenisiert. Der Puffer ist eine Modifikation des von Herrera-Estrella, L., DeBlock, M., Messens, E., Hernalsteens,
J.-P., Van Montagu, M. und J. Schell, EMBO J. 2, 987-995 (1983) verwendeten Puffers, in welchem Albumin aus Rinderblut weggelassen und 0,1 M Sucrose hinzugefügt worden sind. Die Extrakte werden 5 Minuten lang bei 12'000 g zentrifugiert und die überstehende Phase mit Bromphenolblau bis zu einer Endkonzentration von 0,004 % versetzt. Durch Elektrophorese in einem 10 %-igen, nicht denaturierenden Polyacrylamid-Gel werden die Proteine aus 35 µl der überstehenden Phase separiert. Das Gel wird mit einem Kanamycin und γ-³² P-markierte ATP enthaltenden Agarose-Gel überschichtet, inkubiert, und die phophorylierten Reaktionsprodukte werden auf Whatman-p81-Phosphozellulose-Papier übertragen. Das Papier wird sechsmal mit deionisiertem Wasser von 90°C gewaschen und dann autoradiografiert.

Die nachfolgenden Beispiele dienen der näheren Illustration der vorliegenden Erfindung, ohne diese jedoch zu begrenzen. Sie beschreiben die Konstruktion eines Hybridgens und dessen Einbau in Träger-DNS-Sequenzen mit cyclischem Charakter, die Uebertragung dieses Hybridgens in Pflanzenzellen, die Selektionierung der transformierten Pflanzenzellen und die Regeneration von vollständigen Pflanzen aus den transformierten Pflanzenzellen sowie deren kreuzungsgenetische und molekularbiologische Analyse.

In den Beispielen wird das erfindungsgemässe Verfahren wie folgt illustriert:
1) durch Transformation von Tabakpflanzen mittels Uebertragung des NPT-II-Gens, in der Weise, dass man das NPT-II-Gen mit Promoter- und Terminationssignalen des CaMV-Gens VI versieht, dieses in das pUC8-Plasmid einbaut und das erhaltene chimäre Plasmid durch Polyäthylenglykol-Behandlung in isolierte Tabakprotoplasten überträgt,
2) durch Transformation von Pflanzen der Gattung Brassica mittels Uebertragung des NPT-II-Gens, in der Weise, dass man das NPT-II-Gen mit Promoter- und Terminationssignalen des CaMV-Gens VI versieht, diese Konstruktion anstelle des CaMV-Gens VI in das CaMV-Genom einbaut und das erhaltene chimäre Plasmid durch Polyäthylenglykol-Behandlung in isolierte Brassicaprotoplasten überträgt, und
3) durch Transformation von Pflanzen der Gattung Lolium mittels Uebertragung des NPT-II-Gens, in der Weise, dass man das NPT-II-Gen mit Promoter- und Terminationssignalen des CaMV-Gens VI versieht, dieses in das pUC8-Plasmid einbaut und das erhaltene chimäre Plasmid durch Polyäthylenglykol-Behandlung in isolierte Loliumprotoplasten überträgt,

Ferner wird auch die vorteilhafte Beeinflussung der Transformation durch Hitzeschockbehandlung und Elektroporation sowie der kombinierten Methode von Hitzeschockbehandlung, Polyäthylenglykol-behandlung und Elektroporation nach erfolgter Zusammenführung von Protoplasten und NPT-II-Gen durch Beispiele veranschaulicht.

### Beispiel 1: Transformation von Zellen von Nicotiana tabacum c.v. Petit Havana SRI durch Uebertragung des NPT-II-Gens

### a) Konstruktion des Plasmids pABDI

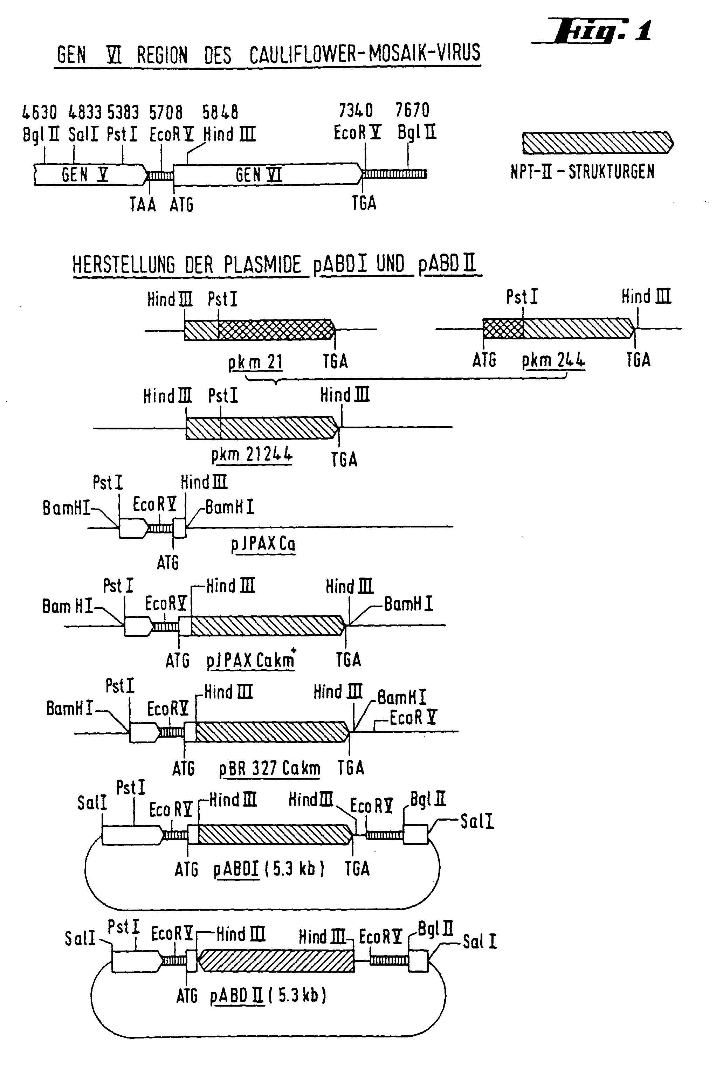

Man zerschneidet die frei zugänglichen Plasmide pkm 21 und pkm 244 (Beck, E., et al., Gene 19, 327-336 (1982)) mit der Restriktions-Endonuclease PstI. Die Fragmente der Plasmide, welche für die Rekombination verwendet werden, werden durch Elektrophorese in 0,8%igem Agarose-Gel gereinigt. Das aus der Verbindung der Bruchstücke erhaltene Plasmid pkm 21244 enthält eine Kombination der 5'-und 3'-Bal 31 - Deletionen des NPT-II-Gens, wie von Beck et al in Gene 19, 327-336 (1982) beschrieben. Um das Promotersignal des Cauliflower-Mosaik-Virus mit dem HindIII-Fragment des Plasmids pkm 21244 zu verbinden, wird das Kupplungsplasmid pJPAX konstruiert. Das Kupplungsplasmid pJPAX wird aus den Plasmiden pUC8 und pUC9 (Messing, J. and J. Vieira, Gene 19, 269-276 (1982)) erhalten. 10 Basenpaare von der Kupplungssequenz des Plasmids pUC9 werden durch Zerschneiden bei der HindIII- und der SalI-Position und nachfolgendem Auffüllen der haftfähigen Enden mit dem Polymerase-I-Klenow-Fragment (Jacobsen, H., et al., Eur. J. Biochem. 45, 623, (1974)) und Verbinden der Polynukleotidkette, wodurch die HindIII-Position wieder hergestellt wird, herausgetrennt. Ein synthetisches Kupplungsglied von 8 Basenpaaren (XhoI) wird an der SmaI-Position dieser abgetrennten Kupplungssequenz eingefügt. Die Rekombination der geeigneten XorI- und HindIII-Fragmente des Plasmids pUC8 und des modifizierten Plasmids pUC9 ergibt das Plasmid pJPAX mit einer teilweise asymmetrischen Kupplungssequenz mit den aufeinander folgenden Restriktionsstellen: EcoRI, SMaI, BamHI, SalI, PstI, HindIII, BamHI, XhoI und EcoRI. Die Verbindung des 5'-Expressionssignals des Cauliflower-Mosaik-Virus-Gens VI und des HindIII-Fragments des NPT-II-Gens wird beim Plasmid pJPAX durch Einfügung der Promoterregion des Cauliflower-Mosaik-Virus-Gens VI zwischen die PstI- und die Hind III-Position bewirkt. Das so erhaltene Plasmid wird an der einzigen HindIII-Position aufgeschnitten und das HindIII-Fragment des Plasmids pkm 21244 wird in diese Schnittstelle in beiden Orientierungsrichtungen eingebaut, wobei man die Plasmide pJPAX Cakm⁺ und pJPAX Cakm⁻ erhält. Um eine EcoRV-Sequenz in der Nähe des 3'Terminationssignals des NPT-II-Hybridgens zu schaffen, wird ein BamHI-Fragment des Plasmids pJPAX Cakm⁺ in die BamHI-Position des Plasmids pBR 327 (Soberon, X. et al., Gene 9, 287-305 (1980)) eingebaut. Man erhält das Plasmid pBR 327 Cakm. Das EcoRV-Fragment des Plasmids pBR 327 Cakm, das die neue DNS-Konstruktion enthält, wird verwendet, um die EcoRV-Region des Cauliflower-Mosaik-Virus-Gens VI zu ersetzen, welches an der SalI-Position im Plasmid pUC8 kloniert ist, wodurch man die Protein-kodierende DNS-Sequenz des NPT-II-Gens unter die Kontrolle der 5'-und 3'-Expressionssignale des Cauliflower-Mosaik-Gens VI stellt. Die so hergestellten Plasmide tragen die Bezeichnung pABDI und pABDII (vgl. Figur 1).

### b) Transformation von Protoplasten von Nicotiana tabacum c.v. Petit Havanna SRI durch Uebertragung des NPT-II-Gens als Teil des Plasmids pABDI mittels PEG-Behandlung.

In 1 ml K₃-Medium [siehe Z. Pflanzenphysiologie 78, 453-455 (1976); Mutation Research 81 (1981) 165-175], enthaltend 0,1 mg/l 2,4-Dichlorphenoxyessigsäure, 1,0 mg/l 1-Naphthylessigsäure und 0,2 mg/l 6-Benzylaminopurin werden in einer Populationsdichte von 2·10⁶ pro ml Tabakprotoplasten suspendiert, die zuvor aus einer Enzymsuspension durch Flotation auf 0,6 molarer Sucrose bei pH 5,8 und anschliessender Sedimentation (100 g für 5 Minuten) in 0,17 molarem Calcium-chlorid bei pH 5,8 gewonnen worden sind. Zu dieser Suspension werden nacheinander 0,5 ml 40%iges Polyäthylenglykol MG 6000 (PEG) in modifiziertem (nach dem Autoklavieren erneut auf pH 5,8 eingestelltem) F-Medium (Nature 296, 72-74 (1982)) und 65 µl einer wässrigen Lösung, enthaltend 15 µg des Plasmids pABDI und 50 µg Kalbsthymus-DNS zugesetzt. Diese Mischung wird unter gelegentlichem Umschwenken für 30 Minuten bei 26°C inkubiert und anschliessend stufenweise mit F-Medium verdünnt. Die Protoplasten werden durch Zentrifugieren (5 Minuten bei 100 g) abgetrennt und in 30 ml frischem K₃-Medium resuspendiert. Die weitere Inkubation erfolgt in 10 ml-Portionen in Petri-Schalen von 10 cm Durchmesser bei 24°C und Dunkelheit, wobei die Populationsdichte 6,3·10⁴ Protoplasten pro ml beträgt. Nach 3 Tagen wird das Kulturmedium pro Schale mit 0,3 Volumenteilen frischem K₃-Medium verdünnt und für weitere 4 Tage bei 24°C und 3000 lux künstlicher Beleuchtung inkubiert. Nach insgesamt 7 Tagen werden die aus den Protoplasten entwickelten Klone in mit 1 % Agarose verfestigtem, 50 mg/l Kanamycin enthaltendem Nährmedium eingebettet und nach der "bead type"-Kulturmethode [Plant Cell Reports, 2, 244-247 (1983)] bei 24°C unter Dunkelheit kultiviert. Das Nährmedium wird alle 5 Tage durch frische gleichartige Nährlösung ersetzt.

### c) Regeneration Kanamycin-resistenter Tabakpflanzen

Nach 3 bis 4 Wochen fortgesetzter Kultivierung in Kanamycin-haltigem Nährmedium werden die resistenten Kalli von 2 bis 3 mm Durchmesser auf mit Agar verfestigtes LS-Kulturmedium [Physiol. Plant 18, 100-127 (1965)], enthaltend 0,05 mg/l 2,4-Dichlorphenoxyessigsäure, 2 mg/l 1-Naphthylessigsäure, 0,1 mg/l 6-Benzylaminopurin, 0,1 mg/l Kinetin und 75 mg/l Kanamycin, verpflanzt. Durch Sprossinduktion auf LS-Medium, enthaltend 150 mg/l Kanamycin und 0,2 mg/l 6-Benzylaminopurin, und anschliessender Wurzelbildung auf T-Medium [Science 163, 85-87 (1969)] erhält man Kanamycin-resistente Nicotiana tabacum-Pflanzen der Sorte Petit Havanna SRI.

### d) Nachweis des NPT-II-Gens im Pflanzenerbgut

0,5 g Kallus der transformierten Zellkulturen oder Blattgewebe der daraus regenerierten Pflanzen werden bei 0°C in 15 %-iger Saccharoselösung, enthaltend 50 mMol/l Aethylendiamin-N,N,N',N'-tetraessigsäure, EDTA), 0,25 Mol/l Natriumchlorid und 50 mMol/l α,α,α-Tris(hydroxymethyl)-methylamin-Hydrochlorid (TRIS-HCl) bei pH 8,0 homogenisiert. Durch Zentrifugieren des Homogenisats für 5 Minuten bei 1000 g trennt man grob die Zellkerne ab. Die Zellkerne werden in 15 %-iger Saccharoselösung, enthaltend 50 mMol/l EDTA und 50 mMol/1 TRIS-HCl, bei pH 8,0 resuspendiert, mit Natrium-dodecylsulfat bis zu einer Endkonzentration von 0,2 % versetzt und für 10 Minuten auf 70°C erhitzt. Nach Abkühlung auf 20-25°C wird der Mischung Kaliumacetat bis zu einer Konzentration von 0,5 Mol/l zugesetzt. Dieses Gemisch wird für 1 Stunde bei 0°C inkubiert. Der ausgefallene Niederschlag wird durch Zentrifugieren (15 Minuten bei 4°C in einer Mikrozentrifuge) zentrifugiert. Aus der überstehenden Flüssigkeit wird durch Versetzen mit dem 2,5-fachen Volumen Aethanol bei 20-25°C die DNS ausgefällt. Die abgetrennte DNS wird in einer Lösung von 10 mMol TRIS-HCl, enthaltend 10 µg/ml Ribonuclease A, gelöst, für 10 Minuten bei 37°C inkubiert, mit Proteinase K bis zu einer Konzentration von 250 µg/ml versetzt und für eine weitere Stunde bei 37°C inkubiert. Die Proteinase K wird durch Phenol- und Chloroform/Isoamylalkohol-Extraktionen enfernt. Aus der wässrigen Phase wird die DNS durch Zusatz von 0,6 Volumenteilen 0,6 molarer isopropanolischer Natriumacetat-Lösung ausgefällt und in 50 µl einer Lösung, enthaltend 10 mMol/l TRIS-HCl und 5 mMol/l EDTA, bei pH 7,5 gelöst. Durch diese Präparation erhält man DNS-Sequenzen, die vorwiegend mehr als 50'000 Basenpaare enthalten. Restriktion dieser DNA mit EcoRV-Endonuclease, Hybridisierung der Fragmente mit radioaktiv markierten HindIII-Bruchstücken des NPT-II-Gens und Vergleich mit dem Plasmid pABDI zeigt in einer "Southern Blot"-Analyse die Anwesenheit des NPT-II-Gens in der Zellkern-DNS der transformierten Nicotiana tabacum-Zellen.

### e) Nachweis der Uebertragung des transformierten Gens auf die sexuellen Nachkommenschaften sowie seiner Vererbung als normales Pflanzengen

Umfangreiche genetische Kreuzungsanalysen und ausführliche molekularbiologische Studien (beispielsweise "Southern blot"-Analyse der DNS des Pflanzengenoms; Untersuchung der Enzymaktivität der Aminoglykosidphosphotransferase, d.h., dem Enzym für die Kanamycinspezifische Phosphorylierung) mit den genetisch veränderten Pflanzen (erste Generation und Nachkommenschaften) haben folgende Ergebnisse erbracht:
1. Das bakterielle Gen ist stabil in das Pflanzengenom eingebaut;
2. Es wird in der Regel unverändert und regelmässig auf Kreuzungsnachkommenschaften übertragen;
3. Sein Erbgang entspricht dem eines natürlichen, einfachen, dominanten Pflanzengens;
4. Die molekulare Analyse mittels DNS-Hybridisierung und Enzymtest bestätigt die Ergebnisse der genetischen Kreuzungsanalyse;
5. Die genetisch veränderten Pflanzen haben ihren normalen, natürlichen Phänotyp während der Behandlung beibehalten; es sind also keine unerwünschten Veränderungen festgestellt worden.

Diese Ergebnisse zeigen, dass mit dem erfindungsgemässen Verfahren des direkten Gentransfers in Protoplasten der beste Weg für die gezielte genetische Veränderung von pflanzlichem Material aufgefunden worden ist. Die genetische Veränderung ist stabil, und unerwünschte Aenderungen des Genotyps der Pflanze treten nicht auf.

Entsprechende Resultate werden bei Durchführung der im vorstehenden Beispiel beschriebenen Transformation auch mit Nicotiana plumbagenifolia, Petunia hybrida, Hyoscyamus muticus und Brassica napus erhalten.

### Beispiel 2: Transformation von Zellen von Brassica rapa c.v. Just Right durch Uebertragung des NPT-II-Gens

### a) Konstruktion des Plasmids pCaMV6km

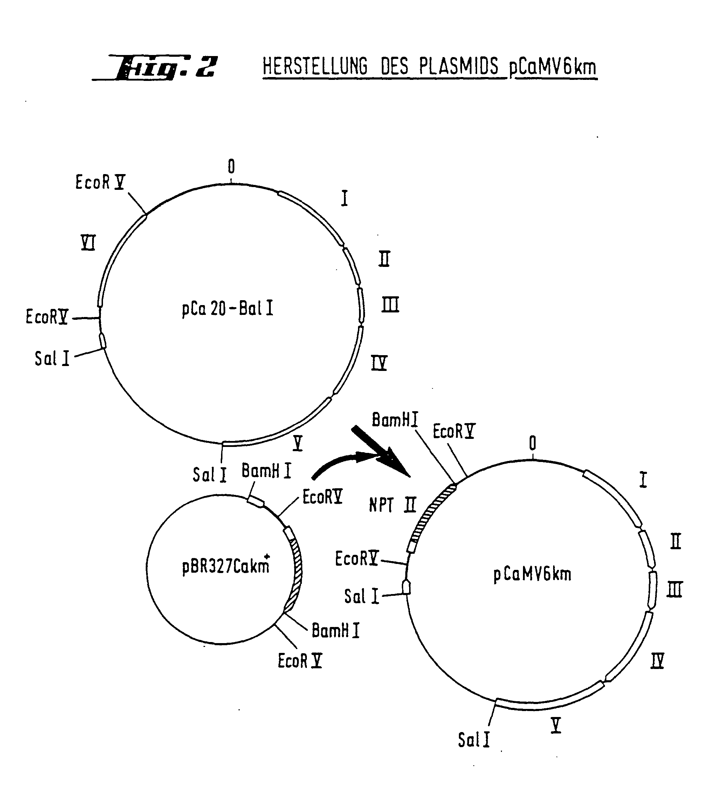

Das unter Beispiel la) beschriebene Plasmid pBR 327 Cakm⁺ wird mit Restriktionsendonuclease EcoRV geschnitten und das EcoRV-Restriktionsfragment mit dem Kanamycin-Resistenz-Gen (NPT-II) gegen das das Gen VI von Cauliflower-Mosaik-Virus enthaltende EcoRV-Fragment des Plasmids pCa20-Bal 1 ausgetauscht. Man erhält so das Plasmid pCaMV6km (Figur 2). Bei dem Plasmid Ca20-Bal 1 handelt es sich um ein chimäres CaMV-Plasmid, welches von der natürlichen Deletionsmutante CM4-184 abstammt. In diesem Plasmid fehlt mit Ausnahme der ersten fünf Codons und dem Translations-Stopsignal TGA die gesamte Region II. Ein XhoI-Kupplungsfragment wurde unmittelbar vor dem Stop-Codon in der Region II eingefügt.

### b) Transformation von Protoplasten von Brassica rapa c.v. Just Right durch Uebertragung des NPT-II-Gens als Teil des Plasmids pCaMV6km mittels PEG-Behandlung

Brassica rapa-Protoplasten werden mit einem geeigneten Osmotikum gewaschen und in einer Populationsdichte von 5 · 10⁶ pro ml in einem Kulturmedium, hergestellt gemäss Protoplasts 83, Poster Proceedings Experientia Supplementum, Birkhäuser Verlag, Basel, Vol. 45 (1983), 44-45, suspendiert. 40 %-iges Polyäthylenglykol MG 6000 (PEG) gelöst in modifiziertem F-Medium (pH 5,8) (vgl. Beispiel 1b) werden mit der Protoplasten-Suspension bis zu einer Endkonzentration von 13 % PEG vermischt. Dieser Mischung wird umgehend eine Lösung von 10 µg mit Endonuclease SalI verdautem Plasmid pCaMV6km und 50 µg Kalbsthymus-DNS in 60 µl Wasser zugesetzt. Unter gelegentlichem Umschwenken wird die Mischung für 30 Minuten bei 20-25°C inkubiert. Anschliessend werden im Abstand von jeweils 5 Minuten dreimal je 2 ml modifiziertes F-Medium (insgesamt 6 ml) und zweimal je 2 ml Kulturmedium (insgesamt 4 ml) zugesetzt. Die so erhaltene Protoplastensuspension wird in Petrischalen von 10 cm Durchmesser überführt und mit weiterem Kulturmedium auf ein Gesamtvolumen von 20 ml aufgefüllt. Diese Protoplastensuspensionen werden bei Dunkelheit für 45 Minuten bei 26°C inkubiert. Die Protoplasten werden durch Sedimentation für 5 Minuten bei 100 g abgetrennt, in ein zunächst flüssiges, dann gelierendes Agarose-Gel-Kulturmedium aufgenommen und nach der "bead type"-Kulturtechnik [Plant Cell Reports 2, 244-247 (1983)] kultiviert. Nach 4 Tagen, im Entwicklungsstadium der ersten Zellteilung, wird den Kulturen Kanamycin in einer Konzentration von 50 mg/l zugesetzt. Das die Agarosesegmente umgebende flüssige Kulturmedium wird alle 4 Tage durch frische, Kanamycin-haltige Nährlösung ersetzt. Nach 4 Wochen isoliert man die Kanamycin-resistenten Klone. Diese werden weitergezüchtet, indem sie wöchentlich mit Kanamycin-haltiger Nährlösung (50 mg/l) versorgt werden.

### c) Nachweis des NPT-II-Gens im Pflanzenerbgut

Durch Isolation der Zellkern-DNS und deren Restriktion und Hybridisierung der DNS-Bruchstücke kann analog zum Beispiel 1d) das Vorhandensein des NPT-II-Gens im Zellkern der transformierten Brassica rapa-Zellen nachgewiesen werden.

### Beispiel 3: Transformation von Gramineenprotoplasten der Spezies Lolium multiflorum

Protoplasten von Lolium multiflorum (Italienisches Raygras) werden in einer Konzentration von 2·10⁶ pro ml in 1 ml 0,4 molarem Mannit, pH 5.8 aufgenommen. Zu dieser Suspension werden nacheinander 0,5 ml 40 %-iges Polyäthylenglykol MG 6000 (PEG) in modifiziertem (pH 5.8) F-Medium (Nature 296, 72-74 (1982), und 65 µl einer wässrigen Lösung, enthaltend 15 µg des Plasmids pABDI und 50 µg Kalbsthymus-DNS zugesetzt. Diese Mischung wird unter gelegentlichem Umschwenken für 30 Minuten bei 26°C inkubiert und anschliessend stufenweise mit F-Medium verdünnt, wie in Nature 296 (1982) 72-74 beschrieben. Die Protoplasten werden durch Zentrifugieren (5 Minuten bei 100 g) abgetrennt und in 4 ml CC-Kulturmedium [Potrykus, Harms, Lörz, Callus formation from cell culture protoplasts of corn (Zea mays L.), Theor. Appl. Genet. 54, 209-214 (1979)] aufgenommen und bei 24°C in Dunkelheit inkubiert. Nach 14 Tagen werden die heranwachsenden Zellkulturen in das gleiche Kulturmedium, jedoch mit dem Antibiotikum G-418 (kommerziell erhältlich; GIBCO EUROPE Produktekatalog, Katalog-Nr. 0661811) übertragen. G-418 ist bei einer Konzentration von 25 mg/Liter toxisch für Loliumzellen und gestattet ausschliesslich die Weiterentwicklung von Zellen, welche das bakterielle Gen für Kanamycinresistenz aufgenommen haben. G-418 ist ein Kanamycin-Analoges mit wesentlich besserer Wirksamkeit in Gramineenzellen, als Kanamycin selbst. Resistente Zellkolonien werden auf Agarmedium (gleiches Medium wie oben, 25 ml/l G-418, ohne Osmotikum) übertragen und nach Erreichen einer Grösse von mehreren Gramm Frischgewicht pro Zellkolonie, auf das Vorliegen des bakteriellen Gens und auf biologische Aktivität des Gens analysiert. Ersteres erfolgt durch Hybridisierung einer radioaktiv markierten DNA-Probe des Gens mit DNA, die aus der Zellkultur isoliert wurde; letzteres geschieht durch Nachweis der Enzymaktivität durch Phosphorylierung von Kanamycin mit radioaktivem ATP. Beide molekularen Nachweisverfahren erbrachten den eindeutigen Beweis für die genetische Transformation der Zellkolonien, die auf G-418 selektioniert worden waren. Die Versuche stellen den erstmaligen Nachweis der genetischen Transformation von Gramineenprotoplasten dar, und beweisen überdies, dass mit dem beschriebenen Verfahren Gräserprotoplasten prinzipiell genetisch manipuliert werden können. Somit ist auch die Möglichkeit der genetischen Manipulation von Kulturgräsern, wie z.B. von Getreide, eröffnet.

### Beispiel 4: Transformation von Zellkultur-Zellen von Nicotiana tabacum durch Uebertragung des NPT-II-Gens unter Anwendung der Elektroporation

Von 50 ml einer log-Phasen-Suspensionskultur der Nitratreduktase-Mangel-Variante von Nicotiana tabacum, Zellstamm nia-115 (Müller, A.J., und R. Grafe, Mol.Gen.Genet. 161, 67-76 (1978)) werden die Protoplasten durch Sedimentation hergestellt, in 20 ml Enzymlösung [2 % Cellulase Onozuka R-10, 1 % Mazerozym R-10 und 0,5 % Driselase (Chemische Fabrik Schweizerhalle, Basel) in Waschlösung (0,3 M Mannit, 0,04 M Calciumchlorid und 0,5 % 2-(N-Morpholino)-äthansulfonsäure); mit KOH auf pH 5,6 eingestellt] resuspendiert und während drei Stunden auf einem Rotationsschüttelapparat bei 24°C inkubiert. Dann werden die Protoplasten durch ein Sieb von 100 um Maschenweite filtriert, wodurch die unverdauten Gewebereste zurückgehalten werden, mit einer volumengleichen Menge 0,6 M Sucrose versetzt und bei 100 g während 10 Minuten zentrifugiert. Die an der Oberfläche flottierenden Protoplasten werden gesammelt und dreimal durch Sedimentation in der Waschlösung gewaschen.

Die Transformation erfolgt unter Anwendung der Elektroporation. Die Kammer vom Dialog^{R} "Porator" (Dialog GmbH, Harffstr. 34, 4000 Düsseldorf, Deutschland-West) wird durch Waschen mit 70 %-igem Aethanol und anschliessendem Waschen mit 100 %-igem Aethanol sterilisiert und zum Trocknen einem sterilen Luftstrom aus einem Gebläse mit laminarer Luftströmung ausgesetzt. Die Protoplasten werden in einer Konzentration von 1 x 10⁶/ml in 0,4 M Mannitol-Lösung suspendiert, mit Magnesiumchlorid auf einen Widerstand von 1,4 kOhm einjustiert und mit pABDI-DNS in einer Konzentration von 10 µg/ml versetzt. Portionen von jeweils 0,38 ml dieser Protoplastensuspension werden 3mal in Abständen von jeweils 10 Sekunden einem Impuls von 1000 Volt oder einem Impuls von 2000 Volt ausgesetzt. Die Protoplasten werden anschliessend in einer Konzentration von 1 x 10⁵/ml in 3 ml AA-CH-Medium (AA-Medium nach Glimelius, K. et al., Physiol. Plant. 44, 273-277 (1978), modifiziert durch Erhöhung des Inosit-Gehaltes auf 100 mg/l und des Saccharose-Gehaltes auf 34 g/l sowie Zusatz von 0,05 ml/l 2-(3-Methyl-2-butenyl)-adenin), welches durch einen Gehalt an 0,6 % Agarose (Sea Plaque, FMC Corp., Marine Colloids Division, P.O. Box 308, Rockland, Maine 04841, USA) in den festen Zustand übergeht, kultiviert. Nach einer Woche wird die die Protoplasten enthaltende Agaroseschicht in 30 ml flüssiges AA-CH-Medium, welches 50 mg/l Kanamycin enthält, überführt. Nach drei Wochen, in denen wöchentlich die Hälfte des flüssigen Mediums durch frisches Medium gleicher Zusammensetzung ersetzt wird, lassen sich die transformierten Zellkolonien optisch wahrnehmen. Diese Zellkolonien werden zur Weiterkultivierung und Untersuchung 4 Wochen, nachdem die Ueberführung in das Kanamycin enthaltende Medium erfolgt ist, auf AA-Medium (Glimelius, K., et al., Physiol. Plant. 44, 273-277 (1978); 0,8 % Agar), welches 50 mg/l Kanamycin enthält, überführt. Die Bestätigung der erfolgreichen Transformation erfolgt durch DNS-Hybridisierung und Prüfung der Enzymaktivität der Aminoglykosidphosphotransferase.

Analoge Versuche mit Protoplasten von Brassica rapa und Lolium multiflorum führen ebenfalls zu erfolgreichen Transformationen.

### Beispiel 5: Transformation von Zellen von Nicotiana tabacum durch Uebertragung des NPT-II-Gens unter Anwendung der Elektroporation

Die Vorbereitung der Elektroporatorkammer erfolgt wie im Beispiel 4 beschrieben und die der Protoplasten wie im Beispiel 1 beschrieben.

Zur Transformation werden Protoplasten von Nicotiana tabacum in einer Konzentration von 1,6 x 10⁶/ml in Mannit-Lösung (0,4 M, gepuffert mit 0,5 Gew. %/Vol. 2-(N-Morpholino)-äthansulfonsäure; pH 5,6) resuspendiert. Der Widerstand der Protoplastensuspension wird in der Poratorkammer (0,38 ml) gemessen und mit Magnesiumchlorid-Lösung (0,3 M) auf 1 bis 1,2 kOhm eingestellt. Portionen von je 0,5 ml werden in verschliessbare Plastikröhrchen (5 ml Volumen) gegeben und pro Röhrchen zunächst 40 µl Wasser, welches 8 µg pABDI (in die lineare Form überführt mit SmaI) und 20 µg Kalbsthymus-DNS enthält, und anschliessend 0,25 ml Poläthylenglykol-Lösung (24 Gew. %/Vol in 0,4 M Mannitol) zugesetzt. 9 Minuten nach Zusatz der DNS werden Portionen von jeweils 0,38 ml in die Impulskammer eingefüllt und 10 Minuten nach der DNS-Zugabe wird die in der Kammer befindliche Protoplastensuspension 3 Impulsen (1000-2000 Volt) in Intervallen von jeweils 10 Sekunden ausgesetzt. Die so behandelten Portionen werden in Petrischalen (Durchmesser 6 cm) gegeben und 10 Minuten lang bei 20°C gehalten. Zu jeder Petrischale werden dann 3 ml K₃-Medium mit einem Gehalt an 0,7 Gew. %/Vol Sea-Plaque-Agarose gegeben und der Inhalt der Schale wird gut vermischt. Nach dem Erstarren des Schaleninhalts werden die Kulturen bei 24°C einen Tag lang bei Dunkelheit, dann 6 Tage bei Licht gehalten. Anschliessend wird die Protoplasten-haltige Agarose in Viertel geschnitten und in flüssiges Medium eingeführt. Die Protoplasten werden nun nach der "bead-type"-Kulturmethode kultiviert. In Kallusgeweben, welche durch Selektionierung des transformierten Materials mittels Kanamycin erhalten werden und in daraus regenerierten Pflanzen ist das NPT-II-Enzym (Aminoglykosidphosphotransferase) als Produkt des NPT-II-Gens vorhanden.

Mit der Elektroporation wird gegenüber der entsprechenden Methode ohne Elektroporation eine Steigerung der Transformationshäufigkeit um das 5- bis 10fache erreicht. Analoge Versuche mit Brassica rapa c.v. Just Right und Lolium multiflorum erbringen ebenfalls eine Steigerung der Transformationshäufigkeit in gleicher Grössenordnung.

### Beispiel 6: Transformation von Zellen von Nicotiana tabacum durch Uebertragung des NPT-II-Gens unter Anwendung der Hitzeschockbehandlung

Protoplasten aus Blättern oder Zellkulturen von Nicotiana tabacum werden isoliert wie in den Beispielen 1 und 4 beschrieben und in ein osmotisches Medium, wie in den vorgängigen Beispielen beschrieben, überführt. Die Protoplastensuspension wird 5 Minuten lang bei einer Temperatur von 45°C gehalten, innerhalb 10 Sekunden mit Eis abgekühlt und anschliessend mit dem Plasmid pABDI versetzt wie in den Beispielen 1 und 4 beschrieben. Die Transformationshäufigkeit wird durch die Hitzeschockbehandlung gegenüber einer Transformation ohne diese Massnahme um einen Faktor von 10 oder grösser gesteigert.

Analoge Versuche mit den in den Beispielen 2 und 3 beschriebenen Protoplasten und Plasmiden ergeben ebenfalls eine Steigerung der Transformationshäufigkeit in gleicher Grössenordnung.

### Beispiel 7: Transformation verschiedener Pflanzenzellen durch Uebertragung des NPT-II-Gens unter Zusammenführung von Protoplasten und Gen als erste Massnahme und anschliessender kombinierter Behandlung

Protoplasten der Pflanzen:
Micotiana tabacum c.v. Petit Havanna SRI (A),
Brassica rapa c.v. Just Right (B) und
Lolium multiflorum (C)
werden isoliert und in osmotisches Medium überführt wie in Beispiel 5 beschrieben. Die Protoplastensuspensionen von A) und C) werden mit dem Plasmid pABDI (Beispiel la), diejenige von B) mit dem Plasmid pCaMV6km (Beispiel 2a) versetzt, wie in den Beispielen 1 bis 3 beschrieben, jedoch ohne gleichzeitige Behandlung mit Polyäthylenglykol. Anschliessend werden die Protoplastensuspensionen einer Hitzeschockbehandlung gemäss Beispiel 6, dann einer Polyäthylenglykolbehandlung wie in den Beispielen 1 bis 3 beschrieben, und anschliessend der Elektroporation gemäss Beispiel 5 unterworfen. Die Transformationshäufigkeit bei diesem Vorgehen liegt im Bereich von 10⁻³ bis 10⁻², wobei je nach Bedingungen 1 bis 2 % erreicht werden können. (Transformationshäufigkeit bei den Beispielen 1 bis 3 bei etwa 10⁻⁵). Ergebnisse im Bereich von 10⁻³ bis 10⁻² werden auch erzielt, wenn nach dem Zusammenbringen von Protoplasten und Plasmiden die nachfolgenden Massnahmen der Hitzeschockbehandlung, der Polyäthylenglykolbehandlung und der Elektroporation in anderer Reihenfolge eingesetzt werden.

## Patentansprüche

1. Transgene Pflanzenzelle, welche eine DNS stabil ins pflanzliche Genom integriert enthält, die ganz oder teilweise heterologen Ursprungs ist im Bezug auf die Rezipientenzelle und die mit Hilfe an sich bekannter mikrobiologischer Verfahren in einer Weise rekombiniert wurde, dass sie im wesentlichen aus einem Strukturgen und flankierenden, zu besagtem Strukturgen heterologen, pflanzenwirksamen Expressionssignalen besteht, mit der Massgabe, dass die integrierte DNS keine die natürlichen pflanzeninfektiösen Eigenschaften bewirkenden Anteile aus pflanzlichen Pathogenen enthält.

2. Transgene Pflanzenzelle gemäss Anspruch 1, dadurch gekennzeichnet, dass es sich bei besagter Pflanzenzelle um eine Zelle aus einer monokotylen Pflanze handelt.

3. Transgene Pflanzenzelle gemäss Anspruch 1, dadurch gekennzeichnet, dass es sich bei besagter Pflanzenzelle um eine Zelle aus einer dikotylen Pflanze handelt.

4. Transgene Pflanzenzelle gemäss Anspruch 2, dadurch gekennzeichnet, dass es sich um eine Zelle aus einer Pflanze der Familie *Gramineae* handelt.

5. Transgene Pflanzenzelle gemäss Anspruch 4, dadurch gekennzeichnet, dass es sich um eine Zelle einer Pflanze ausgewählt aus der Gruppe bestehend aus Mais, Reis, Weizen, Gerste, Roggen, Hafer und Hirse handelt.

6. Transgene Pflanzenzelle gemäss Anspruch 3, dadurch gekennzeichnet, dass es sich um eine Zelle aus einer Pflanze der Familie *Cruciferae* handelt.

7. Transgene Pflanzenzelle gemäss Anspruch 3, dadurch gekennzeichnet, dass es sich um eine Zelle aus einer Pflanze der Familie *Solanaceae* handelt.

8. Transgene Pflanze, die aus Protoplasten regenerierbar ist, sowie deren sexuelle und somatische Nachkommenschaft, welche eine DNS stabil ins pflanzliche Genom integriert enthält, die ganz oder teilweise heterologen Ursprungs ist im Bezug auf die Rezipientenpflanze und die mit Hilfe an sich bekannter mikrobiologischer Verfahren in einer Weise rekombiniert wurde, dass sie im wesentlichen aus einem Strukturgen und flankierenden, zu besagtem Strukturgen heterologen, pflanzenwirksamen Expressionssignalen besteht, mit der Massgabe, dass die integrierte DNS keine die natürlichen pflanzeninfektiösen Eigenschaften bewirkenden Anteile aus pflanzlichen Pathogenen enthält.

9. Transgene Pflanze sowie deren sexuelle und somatische Nachkommenschaft gemäss Anspruch 8, dadurch gekennzeichnet, dass es sich bei besagter Pflanze um eine monokotyle Pflanze handelt.

10. Transgene Pflanze sowie deren sexuelle und somatische Nachkommenschaft gemäss Anspruch 8, dadurch gekennzeichnet, dass es sich bei besagter Pflanze um eine dikotyle Pflanze handelt.

11. Transgene Pflanze sowie deren sexuelle und somatische Nachkommenschaft gemäss Anspruch 9, dadurch gekennzeichnet, dass es sich um eine Pflanze aus der Familie *Gramineae* handelt.

12. Transgene Pflanze gemäss Anspruch 11, dadurch gekennzeichnet, dass es sich um eine Pflanze ausgewählt aus der Gruppe bestehend aus Mais, Reis, Weizen, Gerste, Roggen, Hafer und Hirse handelt.

13. Transgene Pflanze sowie deren sexuelle und somatische Nachkommenschaft gemäss Anspruch 10, dadurch gekennzeichnet, dass es sich um eine Pflanze aus der Familie *Cruciferae* handelt.

14. Transgene Pflanze sowie deren sexuelle und somatische Nachkommenschaft gemäss Anspruch 10, dadurch gekennzeichnet, dass es sich um eine Pflanze aus der Familie *Solanaceae* handelt.

15. Transgene Pflanzenzelle gemäss einem der Ansprüche 1 bis 7, dadurch gekennzeichnet, dass es sich bei besagten pflanzenwirksamen Expressionssignalen um die Expressionssignale des Gens VI des Cauliflower-Mosaik-Virus handelt.

16. Transgene Pflanze sowie deren sexuelle und somatische Nachkommenschaft gemäss einem der Ansprüche 8 bis 14, dadurch gekennzeichnet, dass es sich bei besagten pflanzenwirksamen Expressionssignalen um die Expressionssignale des Gens VI des Cauliflower-Mosaik-Virus handelt.

17. Samen einer transgenen Pflanze gemäss einem der Ansprüche 8 bis 14 sowie 16.

## Claims

1. A transgenic plant cell comprising integrated stably into the plant genome a DNA which is wholly or partly of heterologous origin in respect of the recipient cell and which has been recombined with the aid of conventional microbiological processes such that it consists substantially of a structural gene and flanking expression signals which are heterologous to the said structural gene and have effect in plants, with the proviso that the integrated DNA contains no plant pathogen components which bring about the natural plant-infectious properties.

2. A transgenic plant cell according to claim 1, wherein the said plant cell comprises a cell of a monocotyledonous plant.

3. A transgenic plant cell according to claim 1, wherein the said plant cell comprises a cell of a dicotyledonous plant.

4. A transgenic plant cell according to claim 2, comprising a cell of a plant from the family *Gramineae.*

5. A transgenic plant cell according to claim 4, comprising a cell of a plant selected from the group consisting of maize, rice, wheat, barley, rye, oats and millet.

6. A transgenic plant cell according to claim 3, comprising a cell of a plant from the family *Cruciferae*.

7. A transgenic plant cell according to claim 3, comprising a cell of a plant from the family *Solanaceae.*

8. A transgenic plant regenerable from protoplasts, and its sexual and somatic progeny, comprising integrated stably into the plant genome a DNA which is wholly or partly of heterologous origin in respect of the recipient plant and which has been recombined with the aid of conventional microbiological processes such that it consists substantially of a structural gene and flanking expression signals which are heterologous to the said structural gene and have effect in plants, with the proviso that the integrated DNA contains no plant pathogen components which bring about the natural plant-infectious properties.

9. A transgenic plant and its sexual and somatic progeny according to claim 8, wherein the said plant comprises a monocotyledonous plant.

10. A transgenic plant and its sexual and somatic progeny according to claim 8, wherein the said plant comprises a dicotyledonous plant.

11. A transgenic plant and its sexual and somatic progeny according to claim 9, comprising a plant from the family *Gramineae.*

12. A transgenic plant according to claim 11, comprising a plant selected from the group consisting of maize, rice, wheat, barley, rye, oats and millet.

13. A transgenic plant and its sexual and somatic progeny according to claim 10, comprising a plant from the family *Cruciferae.*

14. A transgenic plant and its sexual and somatic progeny according to claim 10, comprising a plant from the family *Solanaceae.*

15. A transgenic plant cell according to one of claims 1 to 7, wherein the said expression signals having effect in plants comprise the expression signals of the cauliflower mosaic virus gene VI.

16. A transgenic plant and its sexual and somatic progeny according to one of claims 8 to 14, wherein the said expression signals having effect in plants comprise the expression signals of the cauliflower mosaic virus gene VI.

17. Seed of a transgenic plant according to one of claims 8 to 14 and 16.

## Revendications

1. Cellule végétale transgénique, qui contient un ADN intégré d'une manière stable dans le génome végétal, cellule qui est d'origine totalement ou partiellement hétérologue par rapport à la cellule receveuse, et qui a été recombinée, par un procédé microbiologique connu en soi, de telle sorte qu'elle soit constituée pour l'essentiel d'un gène de structure et de signaux d'expression adjacents, hétérologues vis-à-vis du gène de structure mentionné, et actif sur les végétaux, à la condition que l'ADN intégré ne contienne aucune fraction, agissant sur les propriétés naturelles d'infectiosité des végétaux, provenant d'organismes pathogènes végétaux.

2. Cellule végétale transgénique selon la revendication 1, caractérisée en ce que, pour ce qui est de ladite cellule végétale, il s'agit d'une cellule provenant d'une plante monocotylédone.

3. Cellule végétale transgénique selon la revendication 1, caractérisée en ce que, pour ce qui est de ladite cellule végétale, il s'agit d'une cellule provenant d'une plante dicotylédone.

4. Cellule végétale transgénique selon la revendication 2, caractérisée en ce qu'il s'agit d'une cellule provenant d'une plante de la famille des graminées.

5. Cellule végétale transgénique selon la revendication 4, caractérisée en ce qu'il s'agit d'une cellule d'une plante choisie dans l'ensemble comprenant le maïs, le riz, le blé, l'orge, le seigle, l'avoine et le millet.

6. Cellule végétale transgénique selon la revendication 3, caractérisée en ce qu'il s'agit d'une cellule provenant d'une plante de la famille des crucifères.

7. Cellule végétale transgénique selon la revendication 3, caractérisée en ce qu'il s'agit d'une cellule provenant d'une plante de la famille des solanacées.

8. Plante transgénique, qui est régénérable à partir de protoplastes, ainsi que leur descendance sexuelle et somatique, qui contient un ADN intégré d'une manière stable dans le génome végétal, qui est d'origine entièrement ou partiellement hétérologue par rapport à la plante receveuse, et qui a été recombinée à l'aide d'un procédé microbiologique connu en soi, de façon à être constituée pour l'essentiel d'un gène de structure et de signaux d'expression adjacents, hétérologues vis-à-vis dudit gène de structure, actif sur les plantes, à la condition que l'ADN intégré ne contienne pas les fractions d'organismes pathogènes végétaux qui ont une action sur les propriétés naturelles d'infectiosité des plantes.

9. Plante transgénique, ainsi que sa descendance sexuelle et somatique selon la revendication 8, caractérisée en ce que, pour ce qui est de ladite plante, il s'agit d'une plante monocotylédone.

10. Plante transgénique, ainsi que sa descendance sexuelle et somatique selon la revendication 8, caractérisée en ce que, pour ce qui est de ladite plante, il s'agit d'une plante dicotylédone.

11. Plante transgénique, ainsi que sa descendance sexuelle et somatique selon la revendication 9, caractérisée en ce qu'il s'agit d'une plante de la famille des graminées.

12. Plante transgénique selon la revendication 11, caractérisée en ce qu'il s'agit d'une plante choisie dans l'ensemble comprenant le maïs, le riz, le blé, l'orge, le seigle, l'avoine et le millet.

13. Plante transgénique et sa descendance sexuelle et somatique selon la revendication 10, caractérisée en ce qu'il s'agit d'une plante de la famille des crucifères.

14. Plante transgénique et sa descendance sexuelle et somatique selon la revendication 10, caractérisée en ce qu'il s'agit d'une plante de la famille des solanacées.

15. Cellule végétale transgénique selon l'une des revendications 1 à 7, caractérisée en ce que, pour ce qui est desdits signaux d'expression actifs sur les plantes, il s'agit des signaux d'expression du gène VI du virus de la mosaïque du chou-fleur.

16. Plante transgénique et sa descendance sexuelle et somatique selon l'une des revendications 8 à 14, caractérisée en ce que, pour ce qui est desdits signaux d'expression actifs sur les plantes, il s'agit des signaux d'expression du gène VI du virus de la mosaïque du chou-fleur.

17. Semence d'une plante transgénique selon l'une des revendications 8 à 14 et 16.
